(19) Europäisches Patentamt
European Patent Office
Office européen des brevets

(11) Publication number: **0 002 850**
**B1**

(12) **EUROPEAN PATENT SPECIFICATION**

(45) Date of publication of patent specification: **17.02.82**

(21) Application number: **78200351.1**

(22) Date of filing: **06.12.78**

(51) Int. Cl.³: **C 07 C 43/30**, C 07 C 49/703, C 07 C 69/74, C 07 C 49/527

(54) **Novel intermediates in the preparation of cyclopropylcarboxylate esters and process for their manufacture.**

(30) Priority: **16.12.77 GB 5246677**

(43) Date of publication of application:
**11.07.79 Bulletin 79/14**

(45) Publication of the grant of the European patent:
**17.02.82 Bulletin 82/7**

(84) Designated Contracting States:
**BE CH DE FR GB IT LU NL**

(56) References cited:
**FR - A - 2 324 606**
**FR - A - 2 324 607**
**US - A - 4 001 428**

**Chemical Abstracts vol. 75, no. 13, 1971, Columbus, Ohio, U.S.A.**
**G. LAVIELLE et al "Anions associated with the chlorotris (dimethylamino) phosphonium cation. II Synthesis of aliphatic 1,1-dichloro olefins. Reaction mechanism." Page 317, column 1, abstract no. 88029y**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Verbrugge, Pieter Adriaan**
**Badhuisweg 3**
**NL-1031 CM Amsterdam (NL)**
Inventor: **Kramer, Petrus Anthonius**
**Badhuisweg 3**
**NL-1031 CM Amsterdam (NL)**
Inventor: **Van Berkel, Johannes**
**Badhuisweg 3**
**NL-1031 CM Amsterdam (NL)**
Inventor: **Kelderman, Hendrik Cornelis**
**Badhuisweg 3**
**NL-1031 CM Amsterdam (NL)**

(74) Representative: **Keuzenkamp, Abraham et al,**
**P.O. Box 302**
**NL-2501 CH Den Haag (NL)**

**EP 0 002 850 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Chemical Abstracts vol. 88, no. 13, 1978,
Columbus, Ohio, U.S.A.
P. J. V. CLEARE "Halogenated 2-vinyl-1-cyclopropanecarboxaldehydes" page 480,
column 2, abstract no. 89195e

Chemical Abstracts vol. 90, no. 3 1979,
Columbus, Ohio, U.S.A.
P. J. V. CLEARE "Preparation of halogenated aldehydes" page 593, column 1, abstract no. 22 403u

Novel intermediates in the preparation of cyclopropylcarboxylate esters and process for their manufacture

The invention relates to compounds which are useful intermediates in the preparation of cyclopropylcarboxylate esters. The invention also relates to a process for the preparation of these compounds.

The cyclopropylcarboxylate esters are insecticidally-active compounds known as "pyrethroids" and as they combine exceptionally good insecticidal properties with a very low mammalian toxicity. They are of considerable interest to the agrochemical industry and much effort has been expended in finding economic routes to them and to their principal intermediates.

The general formula of one class of these pyrethroid compounds may be represented as follows:—

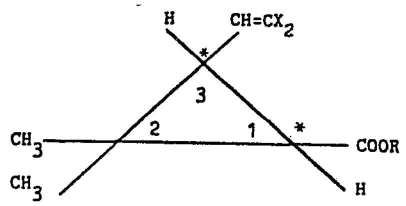

(I)

where each asterisk denotes an asymmetric carbon atom; each X is a halogen atom; and R is a member of a group of radicals known to impart insecticidal activity to the molecule, e.g. 3-phenoxybenzyl or *alpha*-cyano-3-phenoxybenzyl. It is known that the stereoisomeric form of the acid portion of the ester of formula I should be in the (1R, cis) form for maximum insecticidal activity, i.e. the absolute configuration at carbon atom 1 is R and the two hydrogen atoms on carbon atoms 1 and 3 are in a *cis* relationship. This nomenclature is known as the Elliott nomenclature and is defined in M. Elliott, A. W. Farnham, N. F. James, P. H. Needham and D. A. Pullman, Nature, 1974, *248*, 710.

It follows, therefore, that if these stereoisomeric esters of formula I are to be prepared, either a stereospecific chemical route is required or the desired stereoisomer must be obtained from a racemic form by physical separation techniques. The latter are expensive and laborious and not readily employed on an industrial scale. The Applicant has found a stereospecific route which uses as starting material the naturally-occurring substance (+)-3-carene whose formula is as follows:—

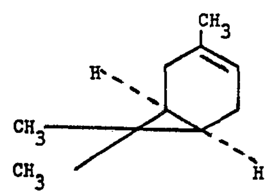

(II)

This compound is an inexpensive readily-available natural terpene and in the present application is disclosed a route to the (1R, cis)-acid portion of the pyrethroid ester of formula I starting from (+)-3-carene and proceeding via a novel cyclopropane compound according to the invention.

The present invention provides compounds of the general formula:—

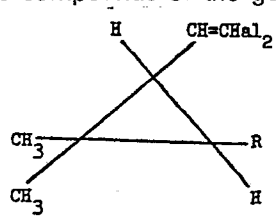

(III)

wherein each Hal represents a halogen atom, especially chlorine or bromine, and R a group selected from:—

(i) 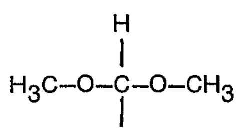

(ii) 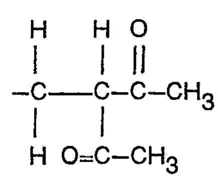

(iii) 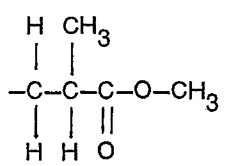

(iv) 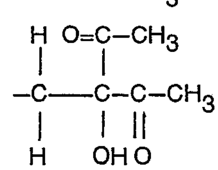

which compounds are in the same stereoisomeric form as that of the cyclopropane ring of (+)-3-carene.

The four groups which R may represent in formula III may be named as follows:—

(i) dimethoxymethyl,
(ii) 2-acetyl-3-oxobutyl,
(iii) 2-methoxycarbonylpropyl and
(iv) 2-acetyl-2-hydroxy-3-oxobutyl

Preferred compounds of the invention of formula III are:—

3 - (2,2 - dichlorovinyl) - 2,2 - dimethylcyclopropane - carbaldehyde dimethyl acetal (compound H);
1 - (2 - acetyl - 3 - oxobutyl) - 2 - (2,2 - dichlorovinyl) - 3,3 - dimethylcyclopropane (compound L);
1 - (2,2 - dichlorovinyl) - 2 - (2 - methoxycarbonylpropyl) - 3,3 - dimethylcyclopropane (compound N); and
1 - (2 - acetyl - 2 - hydroxy - 3 - oxobutyl) - 2 - (2,2 - dichlorovinyl) - 3,3 - dimethylcyclopropane (compound M);

having the same stereoisomeric form as that of the cyclopropane ring (+)-3-carene.

The compounds according to the invention may be prepared by processes known per se, for example, according to methods analogous to those disclosed in UK Patent Specification 1,413,491 which discloses the preparation of dihalovinylcyclopropyl compounds by reacting 3 - formyl - 2,2 - dimethylcyclopropane - carboxylate with a dihalomethylenephosphorane (which can be prepared by reaction of a triorganophosphine, normally triphenylphosphine, with a carbon tetrahalide). The specification discloses an essentially one-step process giving rise to relatively low yields of dihalovinylcyclopropyl compounds (see Examples 15 and 16).

Lavielle *et al* in Bull. Soc. Chim. Fr., No. 6, 1971, Pages 2047 to 2053 described the single step synthesis of dichlorovinyl compounds by reacting together tri(dimethylamino)phosphine, carbon tetrachloride and an aliphatic aldehyde in tetrahydrofuran at a temperature of −78°C. Yields in the range of 50 to 55% are obtained. The use of a temperature as low as −78°C would be a significant disadvantage for a process to be carried out on a commercial scale.

It has been found however that the dihalovinyl compounds of the present invention may advantageously be obtained from the corresponding aldehyde by a two-step process involving the use of a tri(dialkyl)phosphine at relatively accessible temperatures.

Accordingly the present invention also provides a process for the preparation of the compounds of formula III, which process comprises reacting, a tri(dialkylamino)phosphine with a tetrahalomethane and an aldehyde in the presence of a substantially inert solvent, *characterised in that* (a) as a first step the tri(dialkylamino)phosphine is reacted with the tetrahalomethane in the absence of the aldehyde and (b) the product resulting from the first step is reacted with the aldehyde, which has the general formula

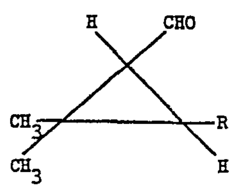

(IV)

having the same stereoisomeric form as that of the cyclopropane ring of (+)-3-carene, wherein R has the same meaning as in formula III, steps (a) and (b) both being effected at temperatures in the range −50°C to +50°C.

Preferably steps (a) and (b) are both effected at temperatures in the range from −20°C to +35°C. The highest yields of the compounds of formula III have been obtained when R in the general formula II represented a dimethoxymethyl group.

The alkyl groups present in the tri(dialkylamino)phosphine may be the same or different and linear or branched. The alkyl groups are suitably the same, have preferably less than six carbon atoms and more preferably less than three. Tri(diethylamino)phosphine and tri(dimethylamino)-phosphine are most preferred.

Tri(dialkylamino)phosphines may be prepared by reaction of a dialkylamine with a phosphorous trihalide, as described in "organic Synthesis," Coll. Vol. V (1973) 602—603. This reaction results in the formation of a solution of the tri(dialkylamino)-phosphine which also contains precipitated dialkylammonium halide. According to a feature of the present invention a tri(dialkylamino)phosphine may be prepared by reacting a dialkylamine with a phosphorous trihalide in the presence of a

substantially-inert solvent. The resulting reaction mixture can then be washed with water to remove unwanted by-products (whether or not after prior separation of the precipitated dialkylammonium halide) and the tri(dialkylamino)phosphine dissolved in the washed solution reacted with the halomethane. It is not necessary to separate the precipitated dialkylammonium halide prior to washing, because this salt is water-soluble. The yield of the compound of formula III can be further enhanced by drying the washed liquid, for example, over a solid drying agent such as anhydrous sodium sulphate or anhydrous magnesium sulphate.

Another attractive feature of the process according to the present invention is that it may be carried out in the presence of an alkane solvent, for example, alkane solvents with a boiling point or boiling range up to 200°C. This also applies to the said reaction between a dialkylamine and a phosphorous trihalide. Examples of alkane solvents are pentane, hexane, heptane, octane and nonane. Mixtures of alkanes are very suitable, for example, gasolines having a boiling range from 62°C to 82°C or from 80°C to 110°C. If desired, the process may be carried out in substantially inert solvents other than alkanes, for example, in tetrahydrofuran.

Examples of tetrahalomethanes are carbon tetrachloride, carbon tetrabromide, carbon tetraiodide, bromotrichloromethane (forming dichlorocarbene) and dibromodifluoromethane (forming difluorocarbene). Very good results have been obtained with carbon tetrachloride. These halomethanes may also act as solvent or cosolvent for the process according to the invention.

The compounds and process according to the invention are of interest as part of a multi-step process to pyrethroid insecticides, e.g. esters based on (1R, cis( - 3 - (2,2 - dichlorovinyl) - 2,2 - dimethyl - cyclopropyl carboxylic acid. An example of such a multi-step route is schematically given below:

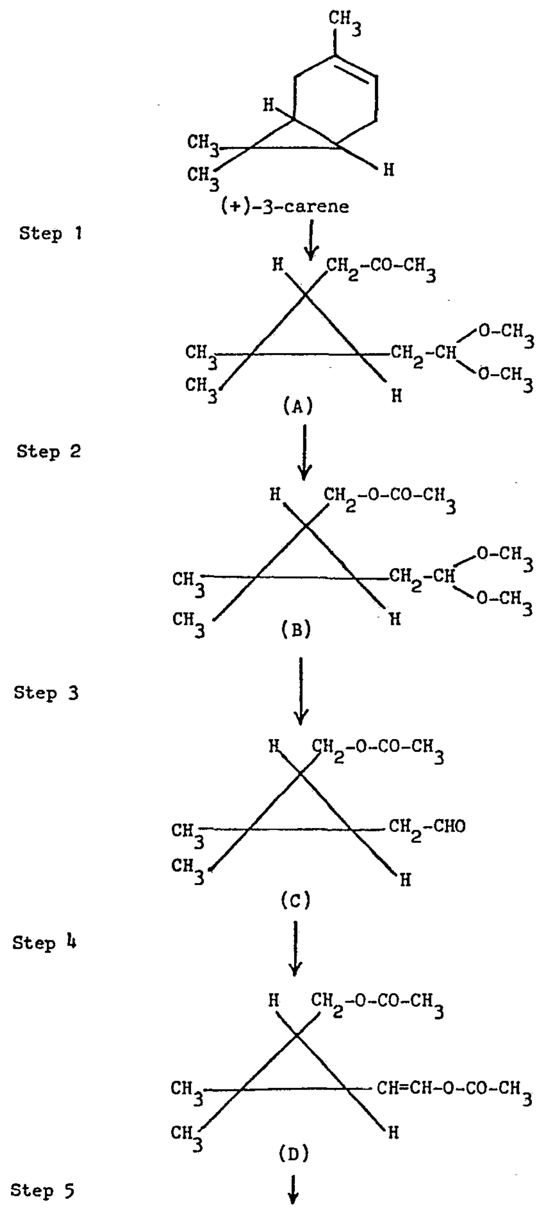

(+)-3-carene
(A)
(B)
(C)
(D)

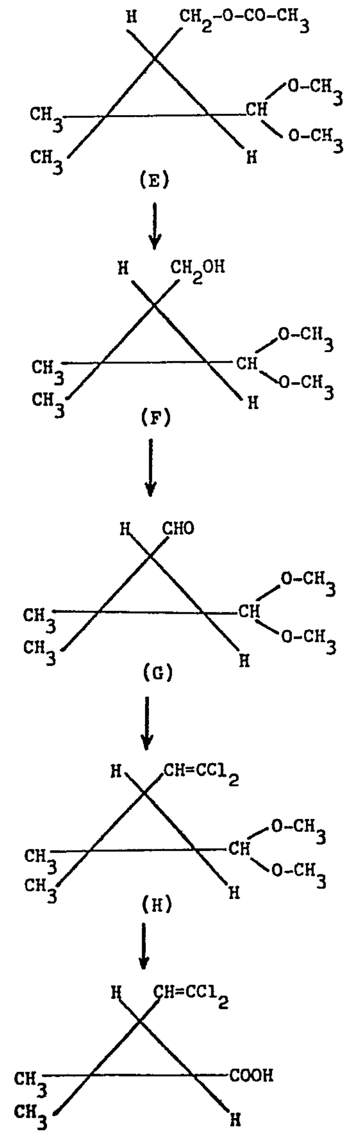

(E)

Step 6

(F)

Step 7

(G)

Step 8

(H)

Step 9

(compare Formula I: R = H; Hal = Cl)

Details of this multi-step process are given below.

Step 1

Ozonolysis of (+)-3-carene, followed by reduction of the ozonolysis product formed (for example with dimethyl sulphide) in the presence of methanol and an acetalizing catalyst (for example p-toluenesulphonic acid) yields a stereoisomer of 1 - (2 - dimethoxyethyl) - 2,2 - dimethyl - 3 - (2 - oxopropyl)cyclopropane (compound A). Ozonolysis of organic compounds and reduction of the peroxidic ozonolysis products formed is described in, for example, Chemical Reviews *58* (1958) 925—995.

Step 2

Oxidation of compound A with a per-acid in the presence of a solvent yields a stereoisomer of 2-(2-(dimethoxyethyl) - 3,3 - dimethylcyclopropylmethyl acetate (compound B). Oxidation of ketones to esters are described in "Methoden der Organischen Chemie," Volume VIII (1952) 559—560. Examples of suitable per-acids are perbenzoic acid, 3-chloroperbenzoic acid and peracetic acid.

Step 3

Hydrolysis of compound B in the presence of, for example acetic acid, yields a stereoisomer of 2,2 - dimethyl - 3 - (2 - formylethyl) - cyclopropyl methyl acetate (compound C). Hydrolysis of acetals to aldehydes is described in "Methoden der Organischen Chemie" (Houben-Weyl), Volume VII, part 1 (1954) 423—428.

Step 4

Reaction of compound C with acetic anhydride in the presence of an amine, for example triethylamine or of an acetate of a strong base, for example sodium acetate, yields a stereoisomer of 2 - (3 - acetoxymethyl - 2,2 - dimethylcyclopropyl)vinyl acetate (compound D). Preparation of enol acetates from anhydrides is described in "Methoden der Organischen Chemie" (Houben-Weyl), Volume VIII (1952) 552.

Step 5

Ozonolysis of compound D, followed by reduction of the ozonolysis product formed (for example with dimethyl sulphide) in the presence of methanol and an acetalizing catalyst (for example p-toluenesulphonic acid) yields a stereoisomer of 3 - acetoxymethyl - 2,2 - dimethylcyclopropanecarbaldehyde dimethyl acetal (compound E). Compound E is claimed in European Patent Application Publication No. 0002556.

Step 6

Hydrolysis of compound E yields a stereoisomer of 3 - hydroxymethyl - 2,2 - dimethylcyclopropanecarbaldehyde dimethyl acetal (compound F). Compound F is claimed in European Patent Application Publication No. 0002852. Hydrolysis of esters is described in, for example, "Methoden der Organischen Chemie" (Houben-Weyl), Volume VIII (1952), 418—423 and 638—639.

Step 7

Oxidation of compound F yields a stereoisomer of 3 - formyl - 2,2 - dimethylcyclopropanecarbaldehyde dimethyl acetal (compound G). Compound G is claimed in European Patent Application Publication No. 0002851. The oxidation of primary alcohols to aldehydes is described in, for example, "Methoden der Organischen Chemie," Volume VII, Part 1 (1954) 159—192. The oxidation is suitably carried out with the chromium trioxide-pyridine complex, as described in J. Org. Chem. *35* (1970) No. 11, 4000—4002.

Step 8

Preparation of a stereoisomer of 3 - (2,2 - dichlorovinyl) - 2,2 - dimethylcyclopropanecarbaldehyde dimethyl acetal (compound H) from compound G via the processes described in Examples I or II.

Step 9

Hydrolysis of compound H in the presence of, for example, acetic acid, followed by oxidation with, for example, hydrogen peroxide in alkaline medium, of the aldehyde formed yields a stereoisomer of 2 - (2,2 - dichlorovinyl) - 3,3 - dimethylcyclopropanecarboxylic acid, whose structure was found to be the same as the (IR, cis)-form of the acid. Certain esters of this acid are very active insecticidal compounds.

The starting aldehydes of formula IV in which R represents a 2 - acetyl - 3 - oxobutyl, a 2-methoxycarbonylpropyl or a 2 - acetyl - 2 - hydroxy - 3 - oxobutyl group may be prepared as described in US patent specification 3,708,528, by ozonolysis of 4-acetyl-2-carene followed by reduction of the ozonolysis product formed. It has been found that this procedure not only yields 3 - (2 - acetyl - 3 - oxobutyl) - 2,2 - dimethylcyclopropanecarbaldehyde, but also 3 - (2 - methoxycarbonylpropyl) - 2,2 - dimethylcyclopropanecarbaldehyde and 3 - (2 - acetyl - 2 - hydroxy - 3 - oxobutyl) - 2,2 - dimethylcyclopropanecarbaldehyde, referred to in the Examples as compounds O, K and J respectively.

The following Examples further illustrate the invention. Yields and purities were determined by means of gas-liquid chromatography and nuclear magnetic resonance (NMR) spectroscopy. The NMR data quoted were recorded at 90 MHz using solutions of the compounds in deuterochloroform.

Example I

Preparation of a stereoisomer of 3 - (2,2 - dichlorovinyl) - 2,2 - dimethylcyclopropanecarbaldehyde dimethyl acetal starting from (+)-3-carene (compound H)

(a) *Preparation of compound A*

A flask was charged with (+)-3-carene (375 mmol) and water-free methanol (150 ml) and kept at a temperature of −60°C. Then, a mixture of ozone and oxygen was passed through the liquid in the flask at a rate of 70 l/h (corresponding to 75 mmol of ozone per hour) until the (+)-3-carene was fully converted (5 hours). The reaction mixture formed was allowed to adopt a temperature of 20°C, dimethyl sulphide (750 mmol) and p-toluene-sulphonic acid (1.74 mmol) were added and the mixture formed was stirred for four days at 20°C. At the end of this period the (+) 3-carene was fully converted into compound A. Methanol and dimethyl sulphide were evaporated from the reaction mixture, at a pressure of 24 mbar (40°C), diethyl ether (150 ml) was added to the residue obtained the solution formed was washed with a 5%w aqueous solution of sodium hydrogen carbonate (30 ml), with four 30 ml portions of water, the washed solution was dried over anhydrous magnesium sulphate and the

solvent was evaporated from the dried liquid at a temperature of 30°C and a pressure of 24 mbar to give a residue (68.9 g). This residue was distilled at 83°C/1 mbar to give a fraction consisting of the cis isomer, compound A, yield 73.5%.

(b) *Preparation of compound B*

The contents of a flask charged with compound A (200 mmol) prepared as in (a) in this Example, chloroform (300 ml) and 3-chloroperbenzoic acid (384 mmol) were stirred at 20°C for 24 hours. The precipitate formed was separated by filtration, the filtered precipitate was mixed with n-pentane (150 ml), the mixture was separated by filtration, the combined filtrates were washed with two 50 ml portions of a 5% solution of sodium carbonate in water and with two 50 ml portions of water, the washed liquid was dried over anhydrous magnesium sulphate and the solvent was evaporated from the dried liquid at a temperature of 90°C and a pressure of 20 mbar to give a residue containing compound B in a yield of 97%. The content of compound B in the residue was 92%; only the cis isomer had been formed.

(c) *Preparation of compound C*

The contents of a flask charged with compound B (218 mmol in the residue prepared as described in b), acetic acid (40 ml) and water (20 ml) were stirred at 60°C during 2.5 hours. The solvent was evaporated from the reaction mixture at a temperature of 45°C and a pressure of 24 mbar, the residue obtained was taken up in diethyl ether (150 ml), the solution obtained was washed with two 50 ml portions of a 5%w solution of sodium hydrogen carbonate in water and with two 50 ml portions of water, the washed solution was dried over anhydrous magnesium sulphate and the solvent was evaporated from the dried liquid at a temperature of 30°C and a pressure of 24 mbar to give a residue containing compound C in a yield of 80%; the content of compound C in the residue was 85%. Only the cis isomer had been formed.

(d) *Preparation of compound D*

The contents of a flask charged with compound C (175 mmol in the residue prepared as described in c), triethylamine (386 mmol) and acetic anhydride (350 ml) were stirred at 20°C for 18 hours. The solvent was evaporated from the reaction mixture at a temperature of 50°C and a pressure of 20 mbar, the residue obtained was taken up in diethyl ether (150 ml), the solution obtained was washed with five 40 ml portions of water, the washed solution was dried over anhydrous magnesium sulphate and the solvent was evaporated from the dried liquid at a temperature of 40°C and a pressure of 20 mbar to give a residue containing compound D in quantitative yield. The content of compound D in the residue was 88.4%. The orientation to the cyclopropane ring was still cis.

(e) *Preparation of compound E*

A flask was charged with compound D (175 mmol, present in the residue obtained in d, water-free methanol (200 ml) and p-toluenesulphonic acid (1.16 mmol) and kept at a temperature of −65°C. Then, a mixture of ozone and oxygen was passed through the liquid in the flask at a rate of 60 l/h (corresponding to 70 mmol of ozone per hour) until compound D was fully converted (2.5 hours). The reaction mixture formed was allowed to adopt a temperature of 20°C, dimethyl sulphide (350 mmol) was added and the mixture formed was stirred for 17 hours at 20°C. Methanol and dimethyl sulphide were evaporated from the reaction mixture, at a pressure of 16 mbar, diethyl ether (50 ml) was added to the residue obtained and so much of a saturated aqueous solution of sodium bicarbonate was added to the mixture that the pH reached a value of 7. Then, the mixture was washed with three 50-ml portions of water, the washed liquid was dried over anhydrous magnesium sulphate and the solvent was evaporated from the dried liquid at a temperature of 40°C and a pressure of 24 mbar to give a residue (29.6 g) containing compound E (yield between 51 and 78%). Only the cis isomer had been formed. The NMR spectrum of compound E showed the following absorptions:

$\delta = 3.38$ ppm (singlet, C—O—$CH_3$)
$\delta = 4.2$ ppm (multiplet, $H$—C—O—$CH_3$)
$\delta = 1.2$ ppm (multiplet, $H$—C—C(H)—$(OCH_3)_2$)
$\delta = 1.18$ ppm (singlet $H_3$C—C—$CH_3$)
$\delta = 1.18$ ppm (singlet, $H_3$C—C—$CH_3$)
$\delta = 1.1$ ppm (multiplet, $H$—C—$CH_2$)
$\delta = 4.2$ ppm (multiplet, H—C—$CH_2$)
$\delta = 2.1$ ppm (singlet, $H_3$C—C(O)—O—)

(f) *Preparation of compound F*

A flask was charged with all of the residue obtained in (e), water (75 ml), sodium hydroxide (150 mmol) and acetone (25 ml) and the liquid obtained was kept under reflux (60°C) for three hours. Then, the acetone and part of the water were evaporated at a pressure of 16 mbar, the residue obtained was extracted with five 50-ml portions of diethyl ether (during the last two extractions so much sodium

chloride was added that the aqueous phase was saturated with this salt), the combined five extract phases were dried over anhydrous magnesium sulphate and the solvent was evaporated from the dried liquid at a temperature of 40°C and a pressure of 24 mbar to give a residue containing compound F in a yield of 51%, calculated on starting compound D. The content of compound F in the residue was 80%. The cis content of compound F was 70%. The NMR spectrum of the cis isomer of compound F showed the following absorptions:

$\delta = 3.32$ ppm (singlet, C—OC$H_3$)
$\delta = 4.83$ ppm (singlet, $H$—C—O—$CH_3$)
$\delta = 1.53$ ppm (doublet, $H$—C—C(H)—$(OCH_3)_2$)
$\delta = 1.05$ ppm (singlet, $H_3$C—C—$CH_3$)
$\delta = 1.05$ ppm (singlet, $H_3$C—C—C$H_3$)
$\delta = 1.2$ ppm (multiplet, $H$—C—$CH_2$)
$\delta = 3.8$ ppm (doublet, $H_2$C—OH)
$\delta = 4.3$ ppm (singlet, $H_2$C—O$H$)

(g) *Preparation of compound G*

A flask was charged with a mixture of pyridine (120 mmol) and methylene chloride (150 ml) and then with chromium trioxide (60 mmol), at a temperature of 20°C. The contents of the flask were stirred for 15 minutes. Then, a solution of 1.74 g of the residue obtained in (f) — which contained 6.39 mmol of compound F — in methylene chloride (5 ml) was added to the contents of the flask and stirring was continued for 20 minutes. The precipitate in the flask was allowed to settle, the liquid in the flask was decanted, the precipitate was washed with three 25-ml portions of diethyl ether, the three washings were filtered over a bed of 2 cm Florisil (trade mark), the combined three filtrates were washed with two 20-ml portions of a 5%w aqueous solution of sodium hydroxide and then with two 20-ml portions of water and the combined washed ethereal liquids were added to the decanted liquid. The liquid thus obtained was dried over anhydrous magnesium sulphate and the solvent was evaporated from the dried liquid at a pressure of 16 mbar to give a residue containing compound G in a yield of 59%, calculated on starting compound F. The content of compound G in the residue was 46.5%. The cis content of compound G was 70%.

The NMR spectrum of the cis isomer of compound G showed the following absorptions:

$\delta = 3.30$ ppm (singlet, C—O—C$H_3$)
$\delta = 4.8$ ppm (doublet, $H$—C—O—$CH_3$)
$\delta = 1.2$ ppm (multiplet, $H$—C—C(H)—$(OCH_3)_2$)
$\delta = 1.22$ ppm (singlet, $H_3$C—C—C—$CH_3$)
$\delta = 1.37$ ppm (singlet, $H_3$C—C—C$H_3$)
$\delta = 1.8$ ppm (doublet, $H$—C—C(O(H)
$\delta = 9.6$ ppm (doublet, $H$—C=O)

(h) *Preparation of compound H*

Tri(dimethylamino)phosphine (48 mmol) was added to a solution of carbon tetrachloride (48 mmol) in pentane (100 ml) at a temperature of 3°C. The mixture obtained was stirred for 10 minutes at a temperature between 3°C and 5°C. This ended the first step. Then, compound G (24 mmol, present in the residue prepared as described in g) was added over a period of 10 minutes, the temperature was allowed to rise to 13°C and stirring was continued for 15 minutes. This ended the second step. The reaction mixture was washed with water (20 ml), the mixture formed was allowed to separate into an aqueous and an organic phase and the organic phase was isolated and dried over anhydrous sodium sulphate. The solvent was evaporated from the dried organic liquid, giving a residue containing compound H in a yield of 77%. The content of compound H in the residue was 47%. The cis content of compound H was 70%. The NMR spectrum of compound·H showed the following absorptions:

$\delta = 3.34$ ppm (singlet, C—O—C$H_3$)
$\delta = 4.3$ ppm (doublet, $H$—C—O—$CH_3$)
$\delta = 1.2$ ppm (multiplet, $H$—C—C(H)—$(OCH_3)_2$)
$\delta = 1.7$ ppm (double doublet, $H$—C—C(H)=C)
$\delta = 5.83$ ppm (doublet, cis $H$—C=C)
$\delta = 5.72$ ppm (doublet, trans $H$—C=C)
cis $\delta = 1.14$ ppm (singlet, $H_3$C—C—$CH_3$); trans $\delta = 1.22$ ppm
cis $\delta = 1.29$ ppm (singlet, $H_3$C—C—C$H_3$); trans $\delta = 1.22$ ppm

Example II

Preparation of compound H using pre-prepared tri(dimethylamino)phosphine

(a) *Preparation of tri(dimethylamino)phosphine*

A solution of dimethylamine (2.22 mol) in pentane (350 ml) was added with stirring over a period

of 20 minutes to a solution of phosphorous trichloride (0.327 mol) in pentane (200 ml) at a temperature of −20°C. This caused the formation of a white precipitate of dimethylamine hydrochloride and a temperature rise to +18°C. The mixture was stirred for 20 hours at 20°C, cooled to 0°C and the suspension was extracted with water (150 ml). The raffinate phase obtained was dried over anhydrous sodium sulphate, the sodium sulphate was filtered off and washed with pentane to give a solution (in total 580 ml) containing tri(dimethylamino)phosphine (yield 70%).

(b) *Preparation of compound H*

The experiment described in section (h) of Example I was repeated but started with the addition of a solution of tri(dimethylamino) phosphine (48 mmol) in pentane (122 ml), which solution was prepared as described in section (a) of Example II, the carbon tetrachloride (48 mmol). The yield of compound H in the residue was again 77%.

Example III

Preparation of compounds L, M and N

(a) *Preparation of a mixture of the compounds I, J and K*

A 100-ml three-necked flask provided with a magnetic stirrer, thermometer, inlet for ozone and a calcium chloride tube was charged with 4-acetyl-2-carene (60 mmol), water-free methanol (1.55 g) and water-free dichloromethane (75 ml) and then with potassium carbonate powder (0.25 g) and kept at a temperature of −70°C. Then, a mixture of ozone and oxygen was passed through the liquid in the flask at a rate of 40 l/h until all of the 4-acetyl-2-carene has been converted (65 min.). Then, dimethyl sulphide (122 mmol) was added, the flask was allowed to adopt a temperature of 20°C and stirring was continued until all the peroxide had reacted away (2.5 hours). The reaction mixture was washed neutral with a saturated aqueous solution of sodium bicarbonate, the organic phase was isolated, the isolated organic phase dried over anhydrous magnesium sulphate and the solvent evaporated from the dried organic liquid, leaving 12.7 g of a residue.

A solution of sulphuric acid (34 mmol) in water (10 ml) was added with stirring to a solution of sodium sulphite 7 aq (68 mmol) in water (40 ml). An amount of 7.1 g of the residue was added to the mixture thus obtained and stirring was continued for 30 minutes at 20°C. The reaction mixture obtained was washed with three 25-ml portions of dichloromethane. A solution of sodium carbonate (40 mmol) in water (20 ml) was added to the washed aqueous solution, which caused the formation of an oily layer. Extraction of the mixture thus obtained with three 25-ml portions of dichloromethane, drying of the combined extract phases over anhydrous magnesium sulphate and evaporation of the solvent from the dried liquid gave 2.9 g of a residue containing the aldehydes O, J and K in a total yield of 22% calculated on starting 4-acetyl-2-carene. The three aldehydes had the cis structure.

(b) *Preparation of compounds L, M and N*

Tri(dimethylamino)phosphine (30 mmol) was added in two minutes to a solution of carbon tetrachloride (30 mmol) in pentane (25 ml) at 20°C, while the flask was cooled with tap water. This addition resulted in a temperature rise to 35°C and in the formation of a white precipitate. The mixture was allowed to adopt a temperature of 20°C. This ended the first step. Subsequently, the aldehyde mixture (2.8 g) prepared in (a) was added in two minutes' time and stirring was continued for 2.5 hours at 20°C. This ended the second step. The reaction mixture was washed with water (50 ml), the organic phase was isolated, washed with three 25-ml portions of water and dried over anhydrous magnesium sulphate. The solvent was evaporated from the dried organic liquid, giving a residue (2.15 g) having the following composition:

| | |
|---|---|
| Compound L | 17%m |
| Compound M | 31%m |
| Compound N | 40%m |
| starting aldehydes | 12%m |

The residue was mixed with a solution of sodium sulphite 7 aq (13 mmol) in water (10 ml) and a solution of sulphuric acid (8 mmol) in water (2.5 ml) and the mixture obtained was stirred for 65 hours. The reaction mixture obtained was extracted with two 25-ml portions of dichloromethane and the combined extract phases were dried over anhydrous magnesium sulphate. The solvent was evaporated from the dried organic liquid, leaving a residue (1.6 g) with the following composition:

| | |
|---|---|
| Compound L | 23%m |
| Compound M | 37%m |
| Compound N | 34%m |
| balance | 6%m |

The residue was subjected to microdistillation to give the following fractions:

| Boiling range, °C | Pressure, mbar | Content of compounds, %m L | M | N |
|---|---|---|---|---|
| 80—95 | 0.3 | 11 | 18 | 71 |
| 106 | 0.5 | 26 | 48 | 26 |
| 106—109 | 0.5 | 35 | 59 | 6 |
| 109 | 0.5 | 34 | 64 | 2 |

The fraction boiling between 80 and 95°C was again subjected to microdistillation to give a fraction consisting of compound N, boiling point 79°C at 0.5 mbar.
The NMR spectra of the three compounds showed the following absorptions:

Compound L
$\delta = 1.02$ ppm (singlet, $H_3C$—C—$CH_3$)
$\delta = 1.13$ ppm (singlet, $H_3C$—C—$CH_3$)
$\delta = 1.55$ ppm (multiplet, CH—$CH_2$)
$\delta = 2.24$ ppm (singlet, $CH_3$—CO)
$\delta = 3.66$ ppm (triplet, O=C—C(H)—C=O)
$\delta = 5.62$ ppm (doublet, $HC{=}CCl_2$)

Compound M
$\delta = 0.99$ ppm (singlet, $H_3C$—C—$CH_3$)
$\delta = 1.15$ ppm (singlet, $H_3C$—C—$CH_3$)
$\delta = 1.55$—2.0 ppm (multiplet, $CH_2$—CH)
$\delta = 2.38$ ppm (singlet, $CH_3$—CO)
$\delta = 5.58$ ppm (doublet, $HC{=}CCl_2$)

The infrared spectrum showed an OH-absorption peak at ca 3460 $cm^{-1}$.

Compound N
$\delta = 1.02$ ppm (singlet, $H_3C$—C—$CH_3$)
$\delta = 1.15$ ppm (singlet, $H_3C$—C—$CH_3$)
$\delta = 1.18$ ppm (doublet, $H_3C$—CH—CO); I = 7 Hz
$\delta = 1.55$ ppm (multiplet, HC—$CH_2$—CH)
$\delta = 2.48$ ppm (multiplet, HC—CO)
$\delta = 3.72$ ppm (singlet, $H_3C$—O—C=O)
$\delta = 5.58$ ppm (doublet, $HC{=}CCl_2$); I = 9 Hz

## Claims

1. A compound of the general formula:

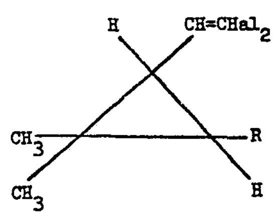

(III)

wherein each Hal is a halogen atom and R is a group selected from:—

(i) 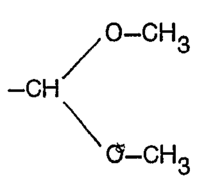

(ii) 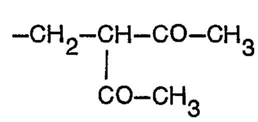

(iii) 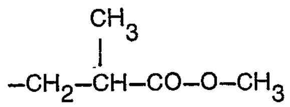, and (iv) 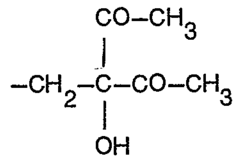

which compound is in the same stereoisomeric form as that of the cyclopropane ring of (+)-3-carene.

2. A compound according to Claim 1 further characterized by having the formula 3-(2,2-dichlorovinyl)-2,2-dimethylcyclopropane-carbaldehyde dimethyl acetal, 1-(2-acetyl-3-oxobutyl)-2-(2,2-dichlorovinyl)-3,3-dimethylcyclopropane, 1-(2,2-dichlorovinyl-2-(2-methoxycarbonylpropyl)-3,3-dimethylcyclopropane, or 1-(2-acetyl-2-hydroxy-3-oxobutyl)-2-(2,2-dichlorovinyl)-3,3-dimethylcyclopropane.

3. A process for the preparation of a compound of formula III as defined in Claim 1 or 2, which process comprises reacting a tri(dialkylamino)phosphine with a tetrahalomethane and an aldehyde in the presence of a substantially inert solvent, characterized in that

(a) as a first step the tri(dialkylamino)phosphine is reacted with the tetrahalomethane in the absence of the aldehyde and

(b) the product resulting from the first step is reacted with the aldehyde, which has the general formula

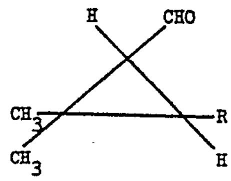
(IV)

having the same stereoisomeric form as that of the cyclopropane ring of (+)-3-carene, wherein R has the same meaning as in formula III, steps (a) and (b) both being effected at temperature in the range −50°C to +50°C.

4. A process according to Claim 3 further characterised in that steps (a) and (b) are both effected at temperatures in the range from −20°C to +35°C.

5. A process according to Claim 3 or 4 further characterised in that the tri(dialkylamino)phosphine is tri(diethylamino)phosphine or tri(dimethylamino)phosphine.

6. A process according to Claim 3, 4 or 5 further characterised in that the substantially-inert solvent is an alkane or tetrahydrofuran.

**Revendications**

1. Composé de la formule générale:

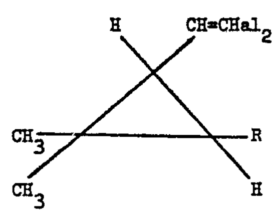
(III)

dans laquelle chaque Hal est un atome d'halogène et R est un groupe choisi parmi:

(i) 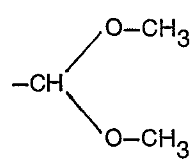 (ii) 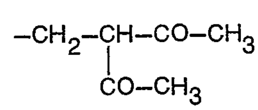

(iii) 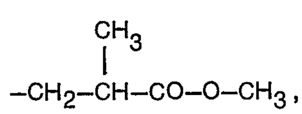, et (iv) 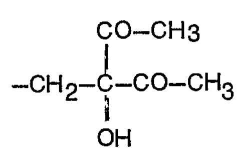

lequel composé est dans la même forme stéréo-isomère que celle du noyau de cyclopropane du (+)-3-carène.

2. Composé selon la revendication 1, caractérisé en ce quil est choisi parmi les suivants: acétal diméthylique de 3-(2,2-dichlorovinyl)-2,2-diméthyl-cyclopropane-carbaldéhyde; 1-(2-acétyl-3-oxobutyl)-2-(2,2-dichlorovinyl)-3,3-diméthyl-cyclopropane; 1-(2,2-dichlorovinyl)-2-(2-méthoxycarbonylpropyl)-3,3-diméthylcyclopropane, ou 1-(2-acétyl-2-hydroxy-3-oxobutyl)-2-(2,2-dichlorovinyl)-3,3-diméthylcyclopropane.

3. Procédé pour la préparation d'un composé de formule III tel que défini dans l'une des revendications 1 et 2, selon lequel on fait réagir une tri(dialcoylamino)phosphine avec un tétrahalométhane et un aldéhyde en présence d'un solvant sensiblement inerte, caractérisé en ce que

(a) comme première étape, la tri(dialcoylamino)phosphine est mise à réagir avec le tétrahalométhane en l'absence de l'aldéhyde et

(b) le produit résultant de la première étape est mis à réagir avec l'aldéhyde, qui a la formule générale:

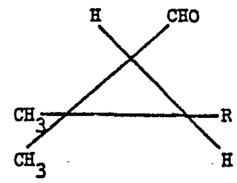

(IV)

ayant la même forme stéréo-isomère que celle du noyau de cyclopropane du (+)-3-carène, où R a la même signification que dans la formule III, les étapes (a) et (b) étant effectuées à des températures comprises entre −50°C et +50°C.

4. Procédé selon la revendication 3, caractérisé en ce que les étapes (a) et (b) sont effectuées toutes deux à des températures comprises entre −20°C et +35°C.

5. Procédé selon l'une des revendications 3 et 4, caractérisé en ce que la tri(dialcoylamino)phosphine est la tri(diéthylamino)phosphine ou la tri(diméthylamino)phosphine.

6. Procédé selon l'une des revendications 3, 4 et 5, caractérisé en ce que le solvant sensiblement inerte est un alcane ou le tétrahydrofuranne.

**Patentansprüche**

1. Verbindung der allgemeinen Formel:

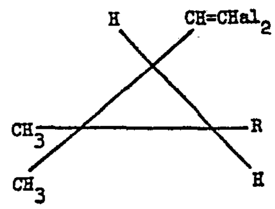

(III)

worin Hal jeweils ein Halogenatom bedeutet und R eine der folgenden Gruppen vertritt:

(1) 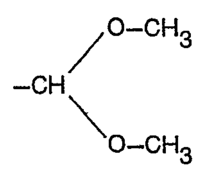 (2) 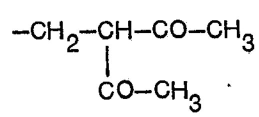

(3) 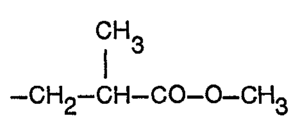 und (4) 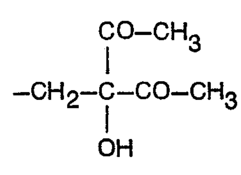

und sie in einer stereoisomeren Form vorliegen, die derjenigen des Cyclopropanringes von (+)-3-Caren entspricht.

2. Verbindung nach Anspruch 1, gekennzeichnet durch eine der folgenden Formeln: 3-(2,2-Dichlorovinyl)-2,2-dimethylcyclopropan-carbaldehyddimethylacetal; 1-(2-Acetyl-3-oxobutyl)-2-(2,2-dichlorvinyl)-3,3-dimethylcyclopropan; 1-(2,2-Dichlorvinyl)-2-(2-methoxycarbonylpropyl)-3,3-dimethylcyclopropan oder 1-(2-Acetyl-2-hydroxy-3-oxobutyl)-2-(2,2-dichlorovinyl)-3,3-dimethylcyclopropan.

3. Verfahren zur Herstellung der Verbindung der Formel III nach Anspruch 1 oder 2 durch Umsetzen eines Tri-(dialkylamino)-phosphins mit einem Tetrahalogenmethan und einem Aldehyd in Gegenwart eines im wesentlichen inertes Lösungsmittels, dadurch gekennzeichnet, dass man

(a) in einer ersten Stufe das Tri-(dialkylamino)-phosphin mit dem Tetrahalogenmethan in Abwesenheit des Aldehyds umsetzt und

(b) das in der ersten Stufe erhaltene Produkt mit dem Aldehyd zur Reaktion bringt, welches der allgemeinen Formel:

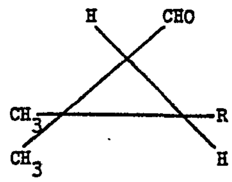

(IV)

entspricht und die gleiche stereoisomere Form hat, die derjenigen des Cyclopropanringes von (+)-3-Caren entspricht, worin R die gleiche Bedeutung wie in Formel III hat und die Stufen a) und b) bei Temperaturen zwischen —50°C und +50°C durchgeführt werden.

4. Verfahren nach Anspruch 3, dadurch gekennzeichnet, dass die Stufen a) und b) jeweils bei einer Temperatur zwischen —20°C und +35°C durchgeführt werden.

5. Verfahren nach Anspruch 3 oder 4, dadurch gekennzeichnet, dass das Tri-(dialkylamino)-phosphin Tri-(diäthylamino)-phosphin oder Tri-(dimethylamino)-phosphin ist.

6. Verfahren nach Anspruch 3, 4 oder 5, dadurch gekennzeichnet, dass das im wesentlichen inerte Lösungsmittel ein Alkan oder Tetrahydrofuran ist.